# EUROPÄISCHE PATENTSCHRIFT

(11) **EP 3 749 636 B1**
(45) Veröffentlichungstag und Bekanntmachung des Hinweises auf die Patenterteilung: **18.10.2023**
(21) Anmeldenummer: 19701248.7
(22) Anmeldetag: 28.01.2019
(51) Int. Cl.: C07C 213/04, C07C 215/08

(54) **VERFAHREN ZUR HERSTELLUNG VON C2-C4-MONOALKANOLAMINEN MITTELS EINES SAUREN KATIONENAUSTAUSCHERS ALS KATALYSATOR**
METHOD FOR THE PREPARATION OF C2-C4-MONOALKANOLAMINES USING AN ACIDIC CATION EXCHANGER AS CATALYST
PROCÉDÉ DE PRODUCTION DE C2-C4-MONOALCANOLAMINES AU MOYEN D'UN ÉCHANGEUR DE CATIONS D'ACIDE EN TANT QUE CATALYSEUR

(30) Priorität: 06.02.2018 EP 18155331
(43) Veröffentlichungstag der Anmeldung: 16.12.2020
(73) Patentinhaber: BASF SE, 67056 Ludwigshafen am Rhein (DE)
(72) Erfinder: GRUENANGER, Christian, 67056 Ludwigshafen (DE); IFFLAND, Gabriele, 67056 Ludwigshafen (DE); KOTANJAC, Zeljko, 67056 Ludwigshafen (DE); LUYKEN, Hermann, 67056 Ludwigshafen (DE); KRUG, Thomas, 67056 Ludwigshafen (DE); YI, Jian Zhong, 67056 Ludwigshaven (DE); MELDER, Johann-Peter, 67056 Ludwigshafen (DE)
(74) Vertreter: BASF IP Association
(86) Internationale Anmeldenummer: PCT/EP2019/051948
(87) Internationale Veröffentlichungsnummer: WO 2019/154647

(56) Entgegenhaltungen:
- DE-A1- 1 941 859

## Beschreibung

Die Anmeldung betrifft ein Verfahren zur kontinuierlichen Herstellung von C₂-C₄-Monoalkanolaminen durch Umsetzung eines entsprechenden C₂-C₄-Alkylenoxids mit einem Überschuss an wässrigem Ammoniak (NH₃) in der Flüssigphase und in Gegenwart eines stark sauren Kationenaustauschers als Katalysator, der ein vernetztes Copolymer als Trägermatrix enthält, das saure funktionelle Gruppen aufweist.

### STAND DER TECHNIK

Für Monoalkanolamine gibt es eine Vielzahl von unterschiedlichen Verwendungsmöglichkeiten. Beispielsweise wird Monoethanolamin (MEOA) als Zusatz in Kühlschmierstoffen, als Zusatz in Reinigungsmitteln und Kosmetika oder als Zwischenprodukt bei der Herstellung von Tensiden eingesetzt. Monoisopropanolamin (MIPOA) kann beispielsweise als Schmierstoff oder als Zwischenprodukt bei der Herstellung von Tensiden eingesetzt werden.

Großtechnisch werden Monoalkanolamine durch Umsetzung von entsprechenden Alkylenoxiden mit Ammoniak hergestellt. Dabei nimmt die Selektivität zum Monoalkanolamin mit steigendem Umsatz ab, weil das Monoalkanolamin mit dem entsprechenden Alkylenoxid weiter zum Dialkanolamin und Trialkanolamin reagiert.

Die Umsetzung der Alkylenoxide mit Ammoniak erfolgt katalytisch. Ein möglicher Katalysator ist Wasser. Höhere Selektivitäten zum Monoalkanolamin lassen sich beispielsweise durch den Einsatz von Zeolithkatalysatoren oder sauren Kationenaustauschern erzielen.

DE 1941859 (Mo Och Domsjö Aktienbolag) offenbart ein Verfahren zur Herstellung von Monoalkanolaminen, wobei saure Kationenaustauscher eingesetzt werden. Das Verfahren kann ferner in Anwesenheit von Wasser durchgeführt werden.

WO 2015/034643 A1, WO 2016/144568 A1 und WO 2017/095687 A1 (alle Dow Global Technologies LLC) beschreiben die Herstellung von sulfonierten Kationenaustauschern auf der Basis von Styrol und Divinylbenzol-haltigen Copolymeren.

Grundsätzlich kann der Einsatz von wässrigem Ammoniak vorteilhaft sein. Bei der Aufarbeitung des Reaktionsgemischs lässt sich bei Anwesenheit von Wasser Ammoniak bei niedrigerem Druck aus dem Reaktionsgemisch entfernen, verglichen mit einer Abtrennung von Ammoniak, ohne dass Wasser in signifikanten Mengen vorhanden ist. In bestehenden Anlagen, bei denen Wasser als Katalysator eingesetzt wird, kann es sich anbieten, zusätzlich einen sauren Kationenaustauscher einzusetzen, um die Effizienz des Prozesses zu steigern. Die Performance eines solchen Kationenaustauschers darf natürlich durch das Vorhandensein von Wasser nicht nachteilig beeinflusst werden.

Der vorliegenden Erfindung lag daher die Aufgabe zugrunde, die Wirtschaftlichkeit bisheriger Verfahren zur Herstellung von Monoalkanolaminen zu verbessern und einem Nachteil oder mehreren Nachteilen des Standes der Technik abzuhelfen. Es sollte also ein Verfahren gefunden werden, in dem wässriger Ammoniak eingesetzt werden kann und das es erlaubt, Monoalkanolaminen mit hohem Umsatz, hoher Ausbeute, Raum-Zeit-Ausbeute [Raum-Zeit-Ausbeuten werden angegeben in Produktmenge / (Katalysatorvolumen • Zeit)' (kg/(I_{Kat.} • h)) und/oder ,Produktmenge / (Reaktorvolumen • Zeit)' (kg/(I_{Reaktor} • h)], und Selektivität herzustellen. Ein solches Verfahren sollte es insbesondere erlauben, Monoalkanolamine mit hohen Ausbeuten herzustellen. Die Ausbeute ergibt sich bekanntlich aus dem Produkt von Umsatz und Selektivität. Eine Verbesserung gegenüber dem Stand der Technik kann also in einer Erhöhung des Umsatzes bei gleichbleibender Selektivität oder auch einer höheren Selektivität bei gleichbleibendem Umsatz bestehen. Besonders erstrebenswert ist natürlich eine gleichzeitige Erhöhung von Umsatz und Selektivität.

Überraschender Weise wurde zur Lösung der oben spezifizierten Aufgabe ein Verfahren zur kontinuierlichen Herstellung von C₂-C₄-Monoalkanolaminen durch Umsetzung eines entsprechenden C₂-C₄-Alkylenoxids mit einem molaren Überschuss an Ammoniak (NH₃), wobei wässriger Ammoniak eingesetzt wird, in der Flüssigphase und in Gegenwart eines sauren Kationenaustauschers als Katalysator, der ein vernetztes Copolymer als Trägermatrix enthält, das saure funktionelle Gruppen aufweist, gefunden, welches dadurch gekennzeichnet ist, dass der Kationenaustauscher eine Gesamtaustauschkapazität von größer oder gleich 1,8 eq/L besitzt, makroporös ist und es sich bei den sauren funktionellen Gruppen um Sulfonsäuregruppen handelt.

### BESCHREIBUNG DER ERFINDUNG

Erfindungsgemäß bedeutet der Begriff "Alkylenoxid", dass sich der Sauerstoff in 1 ,2-Stellung befindet, dass besagte Molekül also eine Epoxidgruppe aufweist. Als solche sind insbesondere Ethylenoxid (EO), Propylenoxid (PO), 1,2-Butylenoxid, 2,3-Butylenoxid und Isobutylenoxid zu nennen. Bevorzugt ist die Herstellung von Monoisopropanolamin (MIPOA) durch Umsetzung von Propylenoxid (PO) und Ammoniak. Ganz besonders bevorzugt ist die Herstellung von Monoethanolamin (MEOA) durch Umsetzung von Ethylenoxid (EO) und Ammoniak.

### Beschreibung des Kationenaustauschers:

Als Katalysator wird ein saurer Kationenaustauscher eingesetzt, der ein vernetztes Copolymer als Trägermatrix enthält, das saure funktionelle Gruppen aufweist. Die Begriffe Trägermatrix oder Matrix können synonym verwendet werden.

Der saure Kationenaustauscher wird üblicherweise in der H⁺-Form eingesetzt. D.h. die sauren funktionellen Gruppen liegen protoniert und nicht etwa als Natrium-Salz oder dergleichen vor. Es ist davon auszugehen, dass aufgrund des vorhandenen Ammoniaks im Verlauf der Reaktion eine Deprotonierung stattfindet und die sauren funktionellen Gruppen dann als entsprechendes Ammonium-Salz vorliegen.

Saure funktionelle Gruppen weisen mindestens ein acides Proton (H⁺) auf. Üblicherweise handelt es sich um Sulfonsäure- (-SO₃H), Carbonsäure- (-CO₂H) oder Phosphorsäuregruppen (-OPO(OH)₂), erfindungsgemäß um Sulfonsäuregruppen und nicht um Carbonsäure- oder Phosphorsäuregruppen.

Die erfindungsgemäße Gesamtaustauschkapazität des Kationenaustauschers bezieht sich auf dessen Zustand vor Inkontaktbringen mit den Edukten (C₂-C₄-Alkylenoxid und wässriger Ammoniak). Sie beträgt bevorzugt 1,8 bis 3,0 eq/L, besonders bevorzugt 1,8 bis 2,5 eq/L und ganz besonders bevorzugt 1,9 bis 2,2 eq/L. Die Gesamtaustauschkapazität in der Einheit eq/L steht für die Stoffmenge (in Mol) an Protonen (H⁺), die in einem Liter (des in der H⁺-Form vorliegenden) Kationenaustauschers gebunden ist. Sie lässt sich gemäß DIN 54403:2009-04 bestimmen. Für die erfindungsgemäßen starksauren Kationenaustauscher (Sulfonsäuregruppen enthaltende Kationenaustauscher) findet das Verfahren A und für schwachsaure, nicht-erfindungsgemäßen Kationenaustauscher (bspw. Carbonsäuregruppen oder Phosphorsäuregruppen enthaltende Kationenaustauscher) findet das Verfahren B der oben benannten DIN Anwendung. Kationenaustauscher wie bspw. der Amberlite 252^{®} H (1,7 eq/L, makroporös, mittelvernetzt) und Amberlite^{®} 131 wet (1,35 eq/L, gelförmig, niedrigvernetzt), beide von der Firma Dow, sind aufgrund ihrer geringen Kationenaustauschkapazität keine erfindungsgemäßen Katalysatoren bzw. Kationenaustauscher.

Die erfindungsgemäßen Kationenaustauscher werden bevorzugt als Kugeln eingesetzt. Der mittlere Durchmesser solcher Kugeln kann 100 bis 1000 µm betragen. Der Durchmesser der Kugeln kann gaußverteilt sein. Bevorzugt ist eine relativ gleichmäßige Größenverteilung ("monodispers"), wobei mindesten 90 Vol.-% der Kugeln einen Durchmesser besitzen, der das 0,9 bis 1,1-fache des mittleren Durchmessers beträgt. Bevorzugt wird der erfindungsgemäße Kationenaustauscher nicht gemahlen.

Ein Kationenaustauscher kann sowohl gelförmig (nicht Teil der beanspruchten Erfindung) als auch makroporös sein. Die erfindungsgemäßen Kationenaustauscher sind makroporös. Die Begriffe gelförmig und makroporös sind dem Fachmann geläufig und beschreiben die Porosität des Kationenaustauschers. Der Begriff "makroporös" beschreibt einen Kationenaustauscher, der sowohl Makroporen als auch Mesoporen besitzt. Mesoporen haben einen Durchmesser von ca. 20 Ä bis ca. 200 Ä (Ä = Angström; 1 Ä = 0,1 nm). Makroporen haben einen Durchmesser von über ca. 200 Ä. Gel-förmige Kationenaustauscher besitzen Poren mit einem Durchmesser von weniger als ca. 20 Ä. Im Gegensatz zu einem gelförmigen Kationenaustauscher haben makroporöse Kationenaustauscher eine permanente poröse Struktur, d.h. auch im trockenen, nicht-gequollenen Zustand. Gel-förmige Kationenaustauscher hingegen müssen erst quellen, damit ihr Inneres für entsprechende Edukte erreichbar ist.

Die makroporösen Kationenaustauscher besitzen bevorzugt eine BET-Oberfläche von 10 bis 100, insbesondere 15 bis 40 m²/g. Die BET-Oberfläche lässt sich mittels N₂-Adsorption am Kationenaustauscher in trockenem Zustand bestimmen.

Die makroporösen Kationenaustauscher haben bevorzugt einen mittleren Porendurchmesser von 100 bis 500 Ä, besonders bevorzugt 150 bis 450 Ä. Der mittlere Porendurchmesser lässt sich mittels N₂-Adsorption am Kationenaustauscher in trockenem Zustand bestimmen.

Das erfindungsgemäße vernetzte Copolymer ist bevorzugt mittel- oder hochvernetzt. Mittelvernetzt sind Copolymere, die einen Vernetzungsgrad von 8 bis 15% aufweisen. Hochvernetzt sind Copolymere die einen Vernetzungsgrad > 15% aufweisen. Niedrigvernetzte Copolymere haben einen Vernetzungsgrad von < 8%. Das erfindungsgemäße vernetzte Copolymer weist bevorzugt einen Vernetzungsgrad von 8 bis 25%, besonders bevorzugt 11 bis 24%, ganz besonders bevorzugt 12 bis 22% auf. Der Vernetzungsgrad ergibt sich aus dem Verhältnis des Gewichts der Monomere, die mindestens eine Verzweigung bilden (bspw. eines Polyvinyliden-Monomers wie Divinylbenzol, Trivinylbenzol oder Ethylenglycoldimethacrylat) zur Gesamtmenge aller Monomere multipliziert mit 100%. Wird beispielsweise zur Herstellung eines Polymers ein Gemisch aus 11 g Divinylbenzol und 89 g Styrol eingesetzt so ergibt sich der Vernetzungsgrad wie folgt: 11 g / (11 g + 89 g) * 100% = 11%.

Ein erfindungsgemäßes vernetztes Copolymer ist bevorzugt herstellbar durch Polymerisation eines Gemischs enthaltend ein aromatisches C₈- bis C₁₆-, bevorzugt C₈- bis C₁₄-Monovinyliden-Monomer sowie ein aromatisches C₁₀- bis C₂₀-, bevorzugt C₁₀- bis C₁₄-Polyvinyliden-Monomer, besonders bevorzugt C₁₀- bis C₁₄-Divinyliden-Monomer.

Ein solches Gemisch kann weitere aliphatische C₃- bis C₁₄- Monomere, die genau eine C=C-Doppelbindung enthalten und/oder aliphatische C₄- bis C₁₄-Monomere, die zwei oder mehrere Doppelbindungen enthalten, umfassen.

Ein Monovinyliden-Monomer besitzt genau eine C=CH₂ Gruppe. Ein Polyvinyliden-Monomer besitz mindestens zwei C=CH₂ Gruppen, ein Divinyliden-Monomer genau zwei C=CH₂ Gruppen. Ein aliphatisches Monomer im Sinne der vorliegenden Erfindung enthält kein aromatisches Ringsystem und kann zudem Heteroatome wie bspw. Sauerstoff (O), Chlor (Cl) oder Brom (Br) enthalten. Ein aromatisches Monomer im Sinne der vorliegenden Erfindung enthält ein aromatisches Ringsystem, das die Hückel-Regel erfüllt und kann zudem Heteroatome wie bspw. die oben genannten enthalten.

Beispiele für aromatische Monovinyliden-Monomere sind Styrol und substituiertes Styrol. Beispiele für substituiertes Styrol sind Vinylnaphthalin, alpha-alkylsubstituiertes Styrol (bspw. alpha-Methylstyrrol), Alkylensubstituiertes Styrol (bspw. Vinyltoluol und Ethylvinylbenzol) sowie halogensubstituiertes Styrol (bspw. Brom. oder Chlorstyrol sowie Vinylbenzylchlorid). Ganz besonders bevorzugt ist Styrrol.

Beispiele für aromatische Divinyliden-Monomere sind Divinylbenzol, Divinyltoluol, Divinylxylen, Divinylnaphthalen, oder Divinyldiphenylsulfon. Ganz besonders bevorzugt ist Divinylbenzol.

Ein Beispiel für ein Polyvinyliden-Monomer ist Trivinylbenzol.

Beispiele für aliphatische Monomere die eine C=C-Doppelbindung enthalten sind Acrylsäure, Methacrylsäure, Methylmethacrylat, Isobornylmethacrylat, Ethylacrylat, Ethylen, Propylen, Acrylnitril, Vinylchloride sowie Mischungen zweier oder mehrerer dieser Monomere. Ein Beispiel für ein aliphatisches Monomer, das zwei C=C-Doppelbindungen enthält, ist Butadien oder Ethylenglykoldimethylacrylat.

Die radikalische Polymerisation der oben beschriebenen Gemische entsprechender Monomere und die anschließende Sulfonierung, beispielsweise mittels konzentrierter Schwefelsäure, Oleum, Chlorsulfonsäure oder Schwefeltrioxid, erfolgt unter den üblichen dem Fachmann bekannten Bedingungen. Die Herstellung von makroporösen Kationenaustauschern ist in UIImanns Encyclopedia of Industrial Chemistry, Vol. A14, 5th Ed., Seite 399, letzter Absatz, und Seite 400 beschrieben.

In einer ganz besonders bevorzugten Ausführungsform ist das erfindungsgemäße Copolymer durch Polymerisation eines Gemischs enthaltend Styrol und Divinylbenzol herstellbar. Besonders bevorzugt besteht das besagte Gemisch aus Styrol und Divinylbenzol. Nach erfolgter Sulfonierung beträgt das molare Verhältnis von Sulfonsäuregruppen zu Phenylresten bevorzugt 1,1 bis 2,0, besonders bevorzugt 1,1 bis 1,5, ganz besonders bevorzugt 1,2 bis 1,4.

Bevorzugte kommerziell erhältliche Kationenaustauscher sind insbesondere Amberlyst^{®} 35 wet (makroporös, hochvernetzt), Amberlyst^{®} 36 wet (makroporös, mittelvernetzt), Amberlyst^{®} 40 wet (makroporös, hochvernetzt) der Firma Dow, oder Lewatit^{®} K2620 der Firma Lanxess.

### Beschreibung des Verfahrens:

Das erfindungsgemäße Verfahren wird kontinuierlich durchgeführt.

Die Umsetzung des C₂-C₄-Alkylenoxids mit Ammoniak erfolgt in der Flüssigphase. Beim Arbeiten in der Flüssigphase leitet man die Edukte simultan in flüssiger Phase, bevorzugt bei den unten genannten Druck- und Temperaturbereichen über den Katalysator.

Der Katalysator ist gewöhnlich als Festbett im Reaktor angeordnet. Bevorzugte Reaktoren sind Rohrreaktoren. Alternativ kann die Umsetzung auch in einem Rohrbündelreaktor oder einer Monostranganlage erfolgen. Der Katalysator wird üblicherweise feucht in den Reaktor eingebaut und anschließend mit Ammoniak gespült.

Bei einer Monostranganlage kann der Rohrreaktor, in dem die Umsetzung erfolgt, aus einer Hintereinanderschaltung mehrerer (z.B. zweier oder dreier) einzelner Rohreaktoren bestehen. Optional ist hier vorteilhaft eine Zwischenspeisung von Feed (enthaltend das Alkylenoxid und/oder den Ammoniak) und/oder Reaktoraustrag aus einem nachgeschalteten Reaktor möglich.

Die Katalysatorbelastung beträgt üblicherweise 0,5 bis 5, bevorzugt 0,8 bis 2 kg Alkylenoxid pro Liter Katalysator und Stunde. Das Volumen des Katalysators bezieht sich auf das Schüttvolumen des feuchten Kationenaustauschers (Katalysators).

Erfindungsgemäß wird wässriger Ammoniak eingesetzt. Dieser weist bevorzugt einen Wassergehalt von 0,1 bis 30 Gew.-% (bezogen auf NH₃) auf. Besonders bevorzugt ist ein Wassergehalt von 0,1 bis 20 Gew.-%, 0,2 bis 19 Gew.-%, 0,5 bis 18 Gew.-%, 0,8 bis 17 Gew.-%, 0,9 bis 16 Gew.-% oder 1 bis 15 Gew.-%, jeweils bezogen auf NH₃. Unter "bezogen auf NH₃" ist zu verstehen, dass sich die Menge an Wasser ausschließlich auf die Menge an NH₃ ohne Berücksichtigung sonstiger Komponenten bezieht. Beispielsweise beträgt in einem Gemisch aus 100 g NH₃, 10 g Wasser und 2 g sonstiger Komponenten der Wassergehalt 10 Gew.-%.

Da das vorhandene Wasser die Reaktion katalysiert ist es zweckmäßig, Ammoniak und Alkylenoxid erst unmittelbar vor dem Inkontaktbringen mit dem Katalysator zu vermischen.

Die Reaktionstemperatur beträgt üblicherweise 30 bis 150°C, bevorzugt 40 bis 140 °C, besonders bevorzugt 40 bis 130°C. Die Reaktion kann unter isothermen oder adiabatischen Bedingungen durchgeführt werden.

Unter isothermen Bedingungen beträgt die Reaktionstemperatur bevorzugt 50 bis 140°C, besonders bevorzugt 60 bis 130 °C. Da die Reaktion stark exotherm ist, muss Wärme von der Reaktionsmischung abgezogen werden, um die Temperatur in etwa konstant zu halten. Dies kann beispielsweise mittels Kühlmänteln erfolgen.

Ebenso kann die Reaktion unter adiabatischen Bedingungen durchgeführt werden. Aufgrund der freigesetzten Reaktionswärme wird die ausgewählte Anfangstemperatur schnell erhöht. Gewöhnlich ist es nicht notwendig, von der Reaktionsmischung Wärme abzuziehen. Bevorzugt beträgt die maximale Temperatur im Reaktor 70 bis 140°C, besonders bevorzugt 100 bis 130°C.

Der Reaktionsdruck ist so zu wählen, dass er größer als der Dampfdruck des jeweiligen Alkylenoxid-, Ammoniak und Wasser enthaltenden Reaktionsgemischs bei der jeweiligen Reaktionstemperatur ist. Dadurch wird erreicht, dass die Edukte in der Flüssigphase vorliegen. Der Reaktionsdruck beträgt üblicherweise 80 bis 150 bar, bevorzugt 90 bis 130 bar.

Der Ammoniak wird im molaren Überschuss eingesetzt. Bevorzugt beträgt das molare Verhältnis von Ammoniak zu Alkylenoxid 5 bis 45. Bei Verhältnissen von kleiner 5 ist die Ausbeute des Monoalkanolamins zu gering. Bei Verhältnissen von über 45 ist keine signifikante Steigerung der Ausbeute mehr zu verzeichnen. Der Einsatz eines höheren Überschusses an Ammoniak und der damit verbundene Mehraufwand bei dessen anschließender Entfernung aus dem Reaktionsgemisch rentieren sich nicht. Besonders bevorzugt beträgt das Verhältnis 7 bis 30, ganz besonders bevorzugt 10 bis 25. Um jeden Zweifel auszuschließen, sei darauf hingewiesen, dass sich der Begriff "molarer Überschuss" bzw. die oben genannten Verhältnisse auf den Ammoniak, ohne Berücksichtigung des erfindungsgemäß vorhandenen Wassers, beziehen.

Nach erfolgter Umsetzung kann das erhaltene Reaktionsgemisch weiter aufgetrennt werden. Dies erfolgt bevorzugt destillativ. Hierbei können zunächst Ammoniak und Wasser bei einem Druck über Normaldruck (1,013 bar) (beispielsweise in einer oder mehreren geeigneten Kolonnen) entfernt werden. Üblicherweise wird der so erhaltene wässrige Ammoniak in die Reaktion zurückgeführt. Das so erhaltene Gemisch besteht überwiegend aus dem entsprechenden Monoalkanolamin, enthält aber üblicherweise auch geringe Mengen an Di- und Trialkanolaminen.

Je nach Verwendungszweck kann ein solches Gemisch direkt eingesetzt werden oder aber es kann destillativ weiter aufgetrennt werden. Dies geschieht üblicherweise unter Zuhilfenahme von geeigneten Kolonnen. Dabei wird das besagte Gemisch einer ersten Kolonne zugeführt, wobei hochreines Monoalkanolamin über Kopf oder einen Seitenabzug abgetrennt wird. Der resultierende Sumpf erhält zu überwiegenden Anteilen Di- und Trialkanolamine. Dieser kann einer zweiten Kolonne zugeführt werden, in welcher hochreines Dialkanolamin über Kopf oder einen Seitenabzug entfernt wird. Das Sumpfprodukt (es enthält überwiegend Trialkanolamin) aus der zweiten Kolonnen kann einer dritten Kolonne zugeführt werden, in welcher hochreines Trialkanolamin über Kopf oder einen Seitenabzug abgetrennt wird. Im Sumpf der dritten Kolonnen fallen etwaige Schwersieder an.

Die folgenden Beispiele dienen der Verdeutlichung der Erfindung, ohne sie in irgendeiner Weise zu beschränken.

### BEISPIELE

### Beschreibung der Experimente:

Flüssiges Ethylenoxid, flüssiger Ammoniak und Wasser wurden unabhängig voneinander durch HPLC-Pumpen in ein 2 ml großes Rührgefäß (Vormischer) gepumpt und dort bei einer Temperatur von 33°C und einem Druck von 100-110 bar vermischt. Die Zulaufmengen und damit die Mengenverhältnisse wurden durch die HPLC-Pumpen gesteuert. Der Druck von 100-110 bar stellte sicher, dass ein homogenes flüssiges Gemisch vorlag. Aus dem Rührgefäß gelangte das Reaktionsgemisch bei 100-110 bar in ein beheiztes Reaktorrohr mit einem Außendurchmesser von 1/8 Zoll, einer Wandstärke von 0,5 mm und einer Länge von 1,35 m. Das Innenvolumen (ca. 5 ml) dieses Reaktorrohres war mit dem entsprechenden Kationenaustauscher gefüllt, wobei sich die Angaben der Belastung auf das Schüttvolumen des eingebauten feuchten Kationenaustauschers beziehen. Das eingefüllte Schüttvolumen wurde bei der Füllung in einem Messzylinder ermittelt. Durch den geringen Durchmesser des Reaktorrohres konnte eine annähernd isotherme Fahrweise sichergestellt werden, was die Vergleichbarkeit der Experimente untereinander gewährleistet. Nach Passieren des Reaktorrohres und einer kurzen Kühlstrecke, wurde das Reaktionsgemisch zur Probennahme durch ein 6-Portventil in wässrige Essigsäure entspannt, was einen sofortigen Stop der Reaktion zur Folge hat (Protonierung des Ammoniaks und Ringöffnung von EO durch Essigsäure zu Glykol bzw. Glycolacetat). Das essigsaure Reaktionsgemisch wurde für die gaschromatographische-Analyse (GC-Analyse) mit Acetanhydrid derivatisiert. Die Produktverteilung von MEOA, DEOA und TEOA (derivatisiert als Acetat) wurde durch eine kalibrierte GC-Methode ermittelt. Hierfür wurde eine 60 m lange GC Säule "RXi-1-ms " verwendet, mit folgendem Temperaturprogramm: Start bei 80 °C, aufheizen mit 8°C/min auf 280°C, 15 Minuten bei 280 °C. Der Umsatz wurde mittels derselben kalibrierten Methode anhand der acetylierten Glykol-Menge (gebildet bei der Essigsäure-Behandlung aus unumgesetzten EO und anschließender Umsetzung mit Acetanhydrid) ermittelt.

Die eingesetzten Kationentaustauscher (alle von der Firma Dow) haben mindestens folgende Gesamtaustauschkapazität.

| | |
|---|---|
| Amberlite 252^{®} H: | 1,7 eg/L (nicht erfindungsgemäß) |
| Amberlite 131^{®} wet: | 1,35 eq/L (nicht erfindungsgemäß) |
| Amberlyst 35^{®} wet: | 1,9 eq/L (erfindungsgemäß) |
| Amberlyst 36^{®} wet: | 1,95 eq/L (erfindungsgemäß) |
| Amberlyst 40^{®} wet: | 2,2 eq/L (erfindungsgemäß) |

Die folgenden Ergebnisse zeigen, dass die erfindungsgemäßen Kationenaustauscher bei verschiedenen Molverhältnissen (Ammoniak zu EO) und unterschiedlichem Wassergehalt des Ammoniaks eine verbesserte Performance im Hinblick auf die Ausbeute liefern.

Die Ergebnisse sind in den Tabellen 1 bis 4 dargestellt.

**Tabelle 1**

| Kat. | T/°C | Katalysatorbelastungkg EO/(I_{Kat} * h) | Molverhältnis (NH₃/EO) | Wassergehalt bezogen auf NH₃/ Gew.-% | Molarer Umsatz bezogen auf EO / % | Produktverteilung / Gew.-% | | | Molare Selektivität (MEOA) / % | Molare Ausbeute (M EOA) / % |
|---|---|---|---|---|---|---|---|---|---|---|
| | | | | | | MEOA | DEOA | TEOA | | |
| Amberlite 252 H (nicht erfindungsgemäß) | 110 | 1,0 | 22 | 15 | 92,0 | 86,2 | 12,6 | 1,2 | 91,7 | 84,4 |
| Amberlite 131 wet (nicht erfindungsgemäß) | 110 | 1,0 | 20 | 15 | 95,9 | 80,9 | 16,8 | 2,3 | 88,3 | 84,7 |
| Amberlyst 36 wet (erfindungsgemäß) | 110 | 1,0 | 22 | 15 | 97,7 | 87,6 | 11,6 | 0,8 | 92,5 | 90,4 |
| Amberlyst 35 wet (erfindungsgemäß) | 110 | 1,0 | 20 | 15 | 95,3 | 87,9 | 11,3 | 0,8 | 92,7 | 88,4 |
| Amberlyst 40 wet (erfindungsgemäß) | 110 | 1,0 | 21 | 15 | 96,0 | 86,6 | 12,3 | 1,1 | 91,9 | 88,3 |

Im Vergleich zum Amberlite 252 H liefern die erfindungsgemäßen Katalysatoren einen höheren Umsatz, ohne dass damit eine Verringerung der Selektivität einhergeht. Dies ist für den Fachmann überraschend. Eigentlich hätte er erwartet, dass bei einer Erhöhung des Umsatzes die Selektivität abnimmt. Im Vergleich zum Amberlite 131 wet liefern die erfindungsgemäßen Katalysatoren bei vergleichbarem Umsatz sogar eine deutlich höhere Selektivität.

**Tabelle 2**

| Kat. | T/°C | Katalysatorbelastung | Molverhältnis (NH₃/EO) | Wassergehalt bezogen auf NH₃/ Gew.-% | Molarer Umsatz bezogen auf EO / % | Produktverteilung / Gew.-% | | | Molare Selektivität (M EOA) / % | Molare Ausbeute (MEOA) / % |
|---|---|---|---|---|---|---|---|---|---|---|
| | | | | | | MEOA | DEOA | TEOA | | |
| | | kg EO/(I_{Kat} * h) | | | | | | | | |
| Amberlite 252 H (nicht erfindungsgemäß) | 110 | 1,0 | 11 | 15 | 99,8 | 70,7 | 23,6 | 5,7 | 81,5 | 81,3 |
| Amberlite 131 wet (nicht erfindungsgemäß) | 110 | 1,0 | 10 | 15 | 99,8 | 63,9 | 26,7 | 9,3 | 76,8 | 76,6 |
| Amberlyst 36 wet (erfindungsgemäß) | 110 | 1,0 | 12 | 15 | 99,8 | 78,0 | 19,2 | 2,8 | 86,4 | 86,2 |
| Amberlyst 35 wet (erfindungsgemäß) | 110 | 1,0 | 11 | 15 | 99,9 | 78,5 | 19,2 | 2,3 | 86,6 | 86,6 |

Durch den Einsatz der erfindungsgemäßen Kationenaustauscher erhält man bei nahezu vollständigem Umsatz eine höhere Selektivität.

**Tabelle 3**

| Kat. | T/°C | Katalysatorbelastung | Molverhältnis (NH₃/EO) | Wassergehalt bezogen auf NH₃/ Gew.-% | Molarer Umsatz bezogen auf EO / % | Produktverteilung / Gew.-% | | | Molare Selektivität (M EOA) / % | Molare Ausbeute (MEOA) / % |
|---|---|---|---|---|---|---|---|---|---|---|
| | | | | | | MEOA | DEOA | TEOA | | |
| | | kg EO/(I_{Kat} * h) | | | | | | | | |
| Amberlite 252 H (nicht erfindungsgemäß) | 110 | 1,0 | 22 | 1 | 84,7 | 87,1 | 12,0 | 0,9 | 92,2 | 78,1 |
| Amberlite 131 wet (nicht erfindungsgemäß) | 110 | 1,0 | 23 | 1 | 72,0 | 86,2 | 12,3 | 1,5 | 91,7 | 66,1 |
| Amberlyst 36 wet (erfindungsgemäß) | 110 | 1,0 | 22 | 1 | 95,2 | 87,0 | 12,1 | 0,9 | 92,2 | 87,7 |
| Amberlyst 35 wet (erfindungsgemäß) | 110 | 1,0 | 20 | 15 | 90,7 | 89,4 | 10,1 | 0,5 | 93,6 | 84,9 |

Die erfindungsgemäßen Katalysatoren liefern einen höheren Umsatz, ohne dass damit eine Verringerung der Selektivität einhergeht. Dies ist für den Fachmann überraschend. Eigentlich hätte er erwartet, dass bei einer Erhöhung des Umsatzes die Selektivität abnimmt.

**Tabelle 4**

| Kat. | T/°C | Katalysatorbelastung | Molverhältnis (NH₃/EO) | Wassergehalt bezogen auf NH₃/ Gew.-% | Molarer Umsatz bezogen auf EO / % | Produktverteilung / Gew.-% | | | Molare Selektivität (M EOA) / % | Molare Ausbeute (MEOA) / % |
|---|---|---|---|---|---|---|---|---|---|---|
| | | | | | | MEOA | DEOA | TEOA | | |
| | | kg EO/(I_{Kat} * h) | | | | | | | | |
| Amberlite 252 H (nicht erfindungsgemäß) | 110 | 1,0 | 11 | 1 | 97,6 | 75,0 | 21,6 | 3,4 | 84,3 | 82,3 |
| Amberlite 131 wet (nicht erfindungsgemäß) | 110 | 1,0 | 11 | 1 | 95,1 | 71,5 | 23,5 | 5,0 | 82,0 | 78,0 |
| Amberlyst 36 wet (erfindungsgemäß) | 110 | 1,0 | 12 | 1 | 99,7 | 77,4 | 19,7 | 2,9 | 86,0 | 85,7 |
| Amberlyst 35 wet (erfindungsgemäß) | 110 | 1,0 | 11 | 1 | 99,6 | 77,5 | 20,2 | 2,3 | 85,9 | 85,6 |

Die erfindungsgemäßen Katalysatoren liefern sowohl eine höhere Selektivität, als auch einen höheren Umsatz.

## Patentansprüche

1. Verfahren zur kontinuierlichen Herstellung von C₂-C₄-Monoalkanolaminen durch Umsetzung eines entsprechenden C₂-C₄-Alkylenoxids mit einem molaren Überschuss an Ammoniak (NH₃), wobei wässriger Ammoniak eingesetzt wird, in der Flüssigphase und in Gegenwart eines sauren Kationenaustauschers als Katalysator, der ein vernetztes Copolymer als Trägermatrix enthält, das saure funktionelle Gruppen aufweist, **dadurch gekennzeichnet, dass** der Kationenaustauscher eine Gesamtaustauschkapazität von größer oder gleich 1,8 eq/L besitzt, makroporös ist und es sich bei den sauren funktionellen Gruppen um Sulfonsäuregruppen handelt.

2. Verfahren nach einem der vorhergehenden Ansprüche, **dadurch gekennzeichnet, dass** die Gesamtaustauschkapazität 1,8 bis 2,5 eq/L beträgt.

3. Verfahren nach einem der vorhergehenden Ansprüche, **dadurch gekennzeichnet, dass** die Gesamtaustauschkapazität 1,9 bis 2,2 eq/L beträgt.

4. Verfahren nach einem der vorhergehenden Ansprüche, **dadurch gekennzeichnet, dass** das vernetzte Copolymer mittelvernetzt oder hochvernetzt ist.

5. Verfahren nach einem der vorhergehenden Ansprüche, **dadurch gekennzeichnet, dass** das vernetzte Copolymer durch Polymerisation eines Gemisches enthaltend ein aromatisches C₈- bis C₁₂-Monovinyliden-Monomer sowie ein aromatisches C₁₀- bis C₁₄-Divinyliden-Monomer herstellbar ist.

6. Verfahren nach dem vorhergehenden Anspruch, **dadurch gekennzeichnet, dass** das vernetzte Copolymer durch Polymerisation eines Gemisches bestehend aus Styrol und Divinylbenzol herstellbar ist.

7. Verfahren nach einem der vorhergehenden Ansprüche, **dadurch gekennzeichnet, dass** der wässrige Ammoniak einen Wassergehalt von 0,1 bis 20 Gew.-% (bezogen auf NH₃) aufweist.

8. Verfahren nach einem der vorhergehenden Ansprüche, **dadurch gekennzeichnet, dass** die Reaktionstemperatur 30 bis 150° C beträgt.

9. Verfahren nach einem der vorhergehenden Ansprüche, **dadurch gekennzeichnet, dass** der Reaktionsdruck 80 bis 150 bar beträgt.

10. Verfahren nach einem der vorhergehenden Ansprüche, **dadurch gekennzeichnet, dass** das molare Verhältnis von Ammoniak zu Alkylenoxid 5 bis 45 beträgt.

11. Verfahren nach einem der vorhergehenden Ansprüche, **dadurch gekennzeichnet, dass** es kontinuierlich durchgeführt wird und der Katalysator im Reaktor als Festbett angeordnet ist.

12. Verfahren nach dem vorhergehenden Anspruch, **dadurch gekennzeichnet, dass** die Katalysatorbelastung 0,5 bis 5 kg Alkylenoxid pro Liter Katalysator und Stunde beträgt.

13. Verfahren nach einem der vorhergehenden Ansprüche zur Herstellung von Monoethanolamin (MEOA) durch Umsetzung von Ethylenoxid (EO) und Ammoniak.

## Claims

1. A process for continuous production of C₂-C₄-monoalkanolamines by reaction of a corresponding C₂-C₄-alkylene oxide with a molar excess of ammonia (NH₃), wherein aqueous ammonia is employed, in the liquid phase and in the presence of an acidic cation exchanger as catalyst which contains a crosslinked copolymer comprising acidic functional groups as carrier matrix, wherein the cation exchanger has a total exchange capacity of not less than 1.8 eq/L, is macroporous and the acidic functional groups are sulfonic acid groups.

2. The process according to any of the preceding claims, wherein the total exchange capacity is 1.8 to 2.5 eq/L.

3. The process according to any of the preceding claims, wherein the total exchange capacity is 1.9 to 2.2 eq/L.

4. The process according to any of the preceding claims, wherein the crosslinked copolymer is moderately crosslinked or highly crosslinked.

5. The process according to any of the preceding claims, wherein the crosslinked copolymer is producible by polymerization of a mixture comprising an aromatic C₈-to C₁₂-monovinylidene monomer and an aromatic C₁₀- to C₁₄-divinylidene monomer.

6. The process according to the preceding claim, **characterized in that** the crosslinked copolymer is producible by polymerization of a mixture consisting of styrene and divinylbenzene.

7. The process according to any of the preceding claims, wherein the aqueous ammonia has a water content of 0.1% to 20% by weight (based on NH₃).

8. The process according to any of the preceding claims, wherein the reaction temperature is 30°C to 150°C.

9. The process according to any of the preceding claims, wherein the reaction pressure is 80 to 150 bar.

10. The process according to any of the preceding claims, wherein the molar ratio of ammonia to alkylene oxide is 5 to 45.

11. The process according to any of the preceding claims, wherein said process is performed as a continuous operation and the catalyst is arranged in the reactor as a fixed bed.

12. The process according to the preceding claim, wherein the catalyst space velocity is 0.5 to 5 kg of alkylene oxide per liter of catalyst per hour.

13. The process according to any of the preceding claims for production of monoethanolamine (MEOA) by reaction of ethylene oxide (EO) and ammonia.

## Revendications

1. Procédé de préparation continue de monoalcanolamines en C₂-C₄ par réaction d'un oxyde d'alkylène en C₂-C₄ correspondant avec un excès molaire d'ammoniac (NH₃), par utilisation d'ammoniaque, en phase liquide et en présence d'un échangeur de cations acide en tant que catalyseur, qui contient un copolymère réticulé en tant que matrice support, qui présente des groupes fonctionnels acides, **caractérisé en ce que** l'échangeur de cations présente une capacité totale d'échange supérieure ou égale à 1,8 éq/l, est macroporeux, et les groupes fonctionnels acides sont des groupes acide sulfonique.

2. Procédé selon l'une des revendications précédentes, **caractérisé en ce que** la capacité totale d'échange est de 1,8 à 2,5 éq/l.

3. Procédé selon l'une des revendications précédentes, **caractérisé en ce que** la capacité totale d'échange est de 1,9 à 2,2 éq/l.

4. Procédé selon l'une des revendications précédentes, **caractérisé en ce que** le copolymère réticulé présente une réticulation moyenne ou élevée.

5. Procédé selon l'une des revendications précédentes, **caractérisé en ce que** le copolymère réticulé peut être fabriqué par polymérisation d'un mélange contenant un monovinylidène en C₈-C₁₂ aromatique monomère, ainsi qu'un divinylidène en C₁₀-C₁₄ aromatique monomère.

6. Procédé selon la revendication précédente, **caractérisé en ce que** le copolymère réticulé est fabriqué par polymérisation d'un mélange consistant en styrène et divinylbenzène.

7. Procédé selon l'une des revendications précédentes, **caractérisé en ce que** l'ammoniaque présente une teneur en eau de 0,1 à 20 % en poids (par rapport au NH₃).

8. Procédé selon l'une des revendications précédentes, **caractérisé en ce que** la température de réaction est de 30 à 150 °C.

9. Procédé selon l'une des revendications précédentes, **caractérisé en ce que** la pression de réaction est de 80 à 150 bar.

10. Procédé selon l'une des revendications précédentes, **caractérisé en ce que** le rapport molaire de l'ammoniac à l'oxyde d'alkylène est de 5 à 45.

11. Procédé selon l'une des revendications précédentes, **caractérisé en ce qu'**il est mis en œuvre en continu, et que le catalyseur est disposé dans le réacteur sous forme d'un lit fixe.

12. Procédé selon la revendication précédente, **caractérisé en ce que** la vitesse spatiale horaire du catalyseur est de 0,5 à 5 kg d'oxyde d'alkylène par litre de catalyseur et par heure.

13. Procédé selon l'une des revendications précédentes, pour la préparation de monoéthanolamine (MEOA) par réaction d'oxyde d'éthylène (OE) et d'ammoniac.
